# NOUVEAU FASCICULE DE BREVET EUROPEEN

(11) **EP 1 543 147 B2**
(45) Date de publication et mention de la décision concernant l'opposition: **16.03.2011**
(45) Mention de la délivrance du brevet: 20.12.2006
(21) Numéro de dépôt: 03773805.1
(22) Date de dépôt: 23.09.2003
(51) Int. Cl.: C12Q 1/14

(54) **PROCEDE DE DETECTION DE STAPHYLOCOCCUS AUREUS RESISTANTS A LA METICILLINE**
VERFAHREN ZUM NACHWEIS VON METHICILLINRESISTENTEN STAPHYLOCOCCUS AUREUS
METHOD OF DETECTING METICILLIN-RESISTANT STAPHYLOCOCCUS AUREUS

(30) Priorité: 23.09.2002 FR 0211718
(43) Date de publication de la demande: 22.06.2005
(73) Titulaire: Rambach, Alain, F-75006 Paris (FR)
(72) Inventeur: RAMBACH, Alain, F-75006 Paris (FR); LE COUSTUMIER, Alain, F-46000 Cahors (FR)
(74) Mandataire: Ahner, Francis
(86) Numéro de dépôt international: PCT/FR2003/002788
(87) Numéro de publication internationale: WO 2004/027086

(56) Documents cités:
- AYERS L W ET AL: "CEFOTETAN A NEW CEPHAMYCIN COMPARISON OF IN-VITRO ANTI MICROBIAL ACTIVITY WITH OTHER CEPHEMS BETA LACTAMASE STABILITY AND PRELIMINARY RECOMMENDATIONS FOR DISC DIFFUSION TESTING" ANTIMICROBIAL AGENTS AND CHEMOTHERAPY, vol. 22, no. 5, 1982, pages 859-877, XP008017736 ISSN: 0066-4804
- JONES R N ET AL: "IN-VITRO ANTI MICROBIAL ACTIVITY EVALUATION OF CEFODIZIME HR-221 A NEW SEMI SYNTHETIC CEPHALOSPORIN" ANTIMICROBIAL AGENTS AND CHEMOTHERAPY, vol. 20, no. 6, 1981, pages 760-768, XP008017735 ISSN: 0066-4804
- KLUYTMANS JAN ET AL: "Performance of CHROMagar selective medium and oxacillin resistance screening agar base for identifying Staphylococcus aureus and detecting methicillin resistance." JOURNAL OF CLINICAL MICROBIOLOGY. UNITED STATES JUL 2002, vol. 40, no. 7, juillet 2002 (2002-07), pages 2480-2482, XP001157563 ISSN: 0095-1137
- DATABASE WPI Section Ch, Week 199511 Derwent Publications Ltd., London, GB; Class B04, AN 1995-077141 XP002243919 & JP 07 000181 A (KYOKUTO SEIYAKU KOGYO KK), 6 janvier 1995 (1995-01-06)
- MERLINO JOHN ET AL: "New chromogenic identification and detection of Staphylococcus aureus and methicillin-resistant S. aureus." JOURNAL OF CLINICAL MICROBIOLOGY, vol. 38, no. 6, juin 2000 (2000-06), pages 2378-2380, XP001157564 ISSN: 0095-1137
- WERTHEIM ET AL: 'Improved detection of methicillin-resistant staphyloccocus aureus ...' J.CLIN.MICROBIOL. vol. 39, no. 7, 01 Janvier 2001, pages 2660 - 2662

## Description

L'invention se rapporte à un nouveau milieu gélifié de détection de *Staphylococcus aureus* résistants à la méticilline, dans lequel sont présents un antibiotique choisi dans le groupe des céphalosporines notamment de seconde ou troisième génération, et un agent chromogène portant un chromophore libéré après hydrolyse avec une enzyme active chez lesdits *Staphylococcus aureus*.

La détection systématique des *Staphylococcus aureus* résistants à la méticilline (aussi écrite méthicilline) (MRSA) est importante.

En effet, bien que les MRSA ne semblent pas plus virulents que les MSSA, (Staphylocoques dorés sensibles à la méticilline), leurs infections sont plus difficiles et onéreuses à traiter. Ceci est dû à ce que la méti-résistance confère la résistance à toutes les béta-lactamines et que cette résistance est très souvent associée à des résistances à de nombreux autres antibiotiques antistaphylococciques majeurs (Lyon et Skurry, Microbiol. Rev. 51 ; 88 - 134)

Pour cette détection on a parfois utilisé des milieux de croissance gélosés supplémentés à la méticilline. Cette méthode est maintenant souvent abandonnée car elle détecte mal certaines souches MRSA, en particulier des souches hétérogènes dans lesquelles des populations bactériennes contiennent une proportion très faible de bactéries franchement résistantes à la méticilline (dans lesquelles 1 bactérie sur 10⁴ ou 10⁸ exprime la résistance). Cette méthode donne un nombre important de résultats faux négatifs.

L'utilisation de milieux de croissance gélosés supplémentés à l'oxacilline est fréquente mais, comme avec la méticilline, certaines souches sont difficiles à détecter.

De plus, pour que ces antibiotiques (méticilline, oxacilline) fonctionnent à peu près efficacement comme suppléments sélectifs, on est contraint de n'employer que certains milieux de croissance. En conséquence les milieux proposés à l'utilisateur sont par exemple des dérivés du Muller Hinton Agar ou du Mannitol Salt Agar. Malheureusement pour l'utilisateur ces milieux sont souvent peu discriminants pour l'espèce *S. aureus* ce qui diminue encore la sensibilité et la spécificité.

On a proposé des milieux de croissance gélosés supplémentés à la tobramycine ou l'ofloxacine, soit dans des bases peu discriminantes (dérivées du Mannitol salt Agar par exemple), soit dans la base très discriminante du CHROMagar Staph aureus, mais la corrélation avec la résistance à la méticilline est médiocre d'où un excès de faux positifs et de faux négatifs.

On utilise aussi la méthode de diffusion sur gélose, par exemple la gélose Mueller Hinton, qui consiste à déposer des disques de papier imprégnés d'antibiotiques sur une gélose ensemencée avec la bactérie à étudier. Il s'établit dans la gélose un gradient de concentration d'antibiotique autour de chaque disque. Après incubation, il se produit un halo d'inhibition autour de chaque disque qui permet de mesurer un diamètre, qui reflète la valeur de la CMI (concentration minimale d'inhibition, concentration minimale pour laquelle aucune croissance des microorganismes n'est détectée).

La présente invention se rapporte à un milieu de culture gélosé permettant de détecter les *Staphylococcus aureus* résistants à la méticilline, avec une bonne sensibilité et une bonne spécificité.

Ainsi, dans le cadre de la présente invention, on utilise les capacités discriminantes des milieux chromogènes, en combinaison avec les propriétés des antibiotiques de la famille des céphalosporines de 2^{éme} ou 3^{ème} génération, qui permettent de détecter les microorganismes résistants à la méticilline.

L'invention se rapporte donc à un milieu de culture gélosé pour la détection de *Staphylococcus aureus* résistants à la méticilline, comprenant, outre des nutriments permettant la croissance desdits *Staphylococcus aureus* à titre d'antibiotique: une céphalosporine choisie dans le groupe constitué de la cefoxitine, le cefmetazole, le moxalactam et le flomoxef et un agent chromogène libérant un chromophore après hydrolyse avec une enzyme active chez lesdits *Staphylococcus aureus.*

Les milieux de culture selon l'invention permettent une détection directe des *Staphylococcus aureus* résistants à la méticilline, en raison de leur croissance sur le milieu selon l'invention et de la présence de(s) l'agent(s) chromogène(s), permettant de définir la nature du microorganisme. On n'a donc pas besoin d'étape supplémentaire de confirmation de la nature des microorganismes poussant sur le milieu selon l'invention.

Les milieux selon l'invention permettent la détection de *Staphylococcus aureus* à la méticilline, à partir d'un inoculum strié sur boîte, alors que la majorité des méthodes de l'art antérieur utilisent un dépôt d'environ 10⁴ à 10⁵ *Staphylococcus aureus* sur la gélose. On peut utiliser les milieux selon l'invention directement à partir d'un prélèvement à partir d'un patient, ou après une phase d'enrichissement.

La céphalosporine utilisée dans la milieu de l'invention est une céphalosporine de seconde ou troisième génération, c'est à dire un antibiotique de la famille des céphalosporines présentant une formule dérivée de la formule (1) suivante : 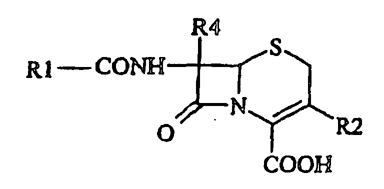 dans laquelle R2 est un groupement H, acétoxy-méthyle, méthyl-thiotétrazole, diméthyl-amino-éthyl-thiotétrazole, triazine, acétamino-pyridine (pyridinium), ou pyridinium substitué par un groupement carbamoyl, cyclo-pento-pyridinium, thiométhyl-acétoxy-thiazole, R1 est un hétérocycle amino-2-thiazole, une α-pipérazine-dione, un α-sulfo-phényle, et R4 est un groupement H ou un radical α-méthoxy.

Cette famille comprend notamment les composés présentant la formule suivante : 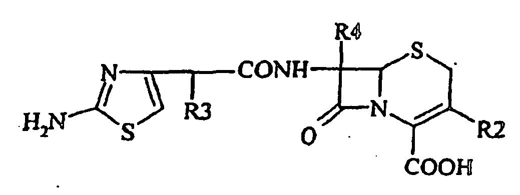 dans laquelle R3 est un groupement H ou un groupement α-méthoxy-imino.

Dans un cas particulier, le groupement R4 est un hydrogène.

Les céphamycines sont des composés dans lesquels le groupement R4 est un radical α-méthoxy, protégeant le noyau β-lactame de l'hydrolyse par les β-lactamases et répondent à la formule suivante :

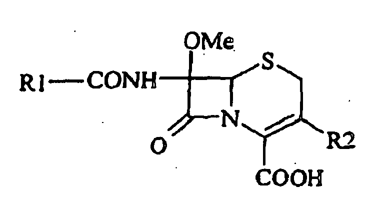

Les oxacephems sont des composés dans lesquels l'atome soufre du noyau céphem est remplacé par un atome d'oxygène, et sont considérés comme dérivés de la formule (I) présentée plus haut.

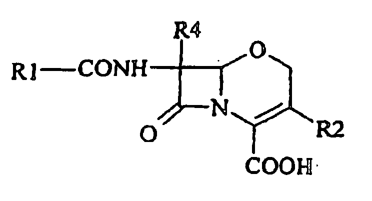

En général, pour ces composés, le groupement R4 est un α-méthoxy.

Une définition des céphalosporines ainsi envisagées peut être trouvée dans Binger (Mécanisme d'Action des Bêta-lactamines, (de la structure bactérienne à la structure de la molécule), 1986 Roussel (Paris) éditeur, chapitre III pages 47-62, et chapitre IV pages 63-68), et dans l'ouvrage de Richmond (Beta-lactam antibiotics (the background to their use as therapeutic agents), Hoechst Aktiengesellschaft, D-6230 Frankfurt (Main) 80 éditeur, 1981, chapitre 3 pages 55-65).

A titre accessoire, on peut noter que les céphalosporines de seconde génération présentent un meilleur effet que les céphalosporines de première génération contre les bactéries Gram négatives et résistent mieux à la dégradation par les β-lactamases, les céphalosporines de troisième génération présentant un spectre d'effet encore plus large vis-à-vis des bactéries Gram négatives.

Parmi les céphalosporines de seconde et troisième génération, on peut citer : loracarbef, cefaclor, cefuroxime, cefprozil, cefoxitin (cefoxitan), cefamandole, cefotian, cefotetan, cefmetazole, cefocinide, ceforanide, cefpodoxime, cefixime, cefotaxime, ceftizoxime, ceftriaxone, ceftazidime, cefmenoxime, cefodizime, cefoperazone, cefepime, cefpirome, cefsulfonide, cefetamete, ceftibutene, moxalactam, latamoxef et flomoxef, notamment sous forme de sels (sodiques). L'homme du métier peut obtenir des listes de tels composés, notamment sur Internet au site www.biam2.org ou www.fpnotebook.com.

La concentration en antibiotique dans le milieu selon l'invention est de préférence comprise entre 0,5 et 50 mg/l, de préférence 1,5 et 30 mg/l, en particulier 1,5 et 15 mg/l. Quelques essais de routine permettent à l'homme du métier de l'ajuster en fonction de la CMI (concentration minimale d'inhibition, concentration minimale pour laquelle aucune croissance des microorganismes n'est détectée) des microorganismes considérés pour l'antibiotique considéré.

Il est à noter que parmi les antibiotiques susceptibles d'entrer dans la composition d'un milieu de l'invention, certains sont capables de conférer certaines propriétés audit milieu. Par exemple, la cefoxitine ou le cefmetazole confèrent une stabilité particulière, d'au moins 2 mois audit milieu.

Par exemple, on a observé une stabilité d'au moins 2 mois pour un milieu conforme à l'invention préparé avec de la cefoxitine à une concentration de 5 mg/l, ajoutée au milieu quand celui-ci est à une température de 48°C. La stabilité du milieu conforme à l'invention a été portée à au moins 5 mois quand celui-ci a été préparé avec du cefmetazole à une concentration de 2,5 mg/l, ajouté au milieu lorsque celui-ci est à une température de 48°C.

Par « stabilité du milieu », on entend désigner la capacité du milieu conforme à l'invention à permettre la détection sélective des *Staphylococcus aureus* résistants à la méticilline, pendant un temps donné, avec la même fiabilité pendant toute la durée de la période.

Il est également à noter que le flomoxef, présente quant à lui la capacité à conserver son activité antibiotique lorsqu'il est soumis à une température supérieure à 100°C (sa résistance ayant même été vérifiée par les inventeurs à 121°C).

Une telle propriété permet de réaliser un milieu conforme à l'invention sous forme d'une unique poudre, ce qui limite considérablement les manipulations nécessaires à effectuer dans des conditions stériles pour la préparation de tout milieu de culture. Le flomoxef peut donc être incorporé au milieu en préparation avant chauffage ce qui en facilite la préparation. Cet avantage n'est pas négligeable car est de nature à faciliter la tâche de toute personne ayant à préparer de tels milieux, quelle que soit son expérience en la matière.

Dans un mode de réalisation préféré, lesdits microorganismes sont des staphylocoques, et on utilise préférentiellement un agent chromogène choisi dans le groupe constitué du 5-bromo 6-chloro 3-indoxyl phosphate et du 5-bromo 4-chloro 3-indoxyl glucoside,ainsi que décrit dans la demande WO 00/53799.

Les milieux de culture de *S. aureus* sont connus et décrits notamment dans le manuel "Oxoïd Unipath Limited", Wade Road, Basingstoke, Hampshire, RG24 OPW, Angleterre. Il peut s'agir, par exemple de "Nutrient Agar Oxoïd CM3", milieu essentiellement à base d'extraits de levure, de peptone et d'agar.

Dans un mode de réalisation préféré, ledit milieu de culture contient à la fois du 5-bromo 6-chloro 3-indoxyl phosphate et du 5-bromo 4-chloro 3-indoxyl glucoside.

Les milieux selon la présente invention contiendront, de préférence, de 0,01 à 0,50 g/l, notamment de 0,05 à 0,40 g/l de 5-bromo 6-chloro 3-indoxyl phosphate, de préférence de 0,01 à 0,20 g/l de 5-bromo 4-chloro 3-indoxyl glucoside, de préférence de 0,01 à 0,20 g/l de 5-bromo 4-chloro 3-indoxyl galactoside, de préférence de 0,01 à 0,20 g/l de 5-bromo 4-chloro 3-indoxyl glucuronide.

Dans un mode de réalisation particulier, le milieu de culture selon l'invention comporte, en outre, au moins l'un des deux agents chromogènes suivants : 5-bromo 4-chloro 3-indoxyl galactoside et 5-bromo 4-chloro 3-indoxyl, glucuronide.

Dans un mode de réalisation particulier, ledit milieu contient en outre de la déféroxamine. La déféroxamine permet en effet d'inhiber *Staphylococcus epidermis* sans inhiber *Staphylococcus aureus,* la concentration mise en oeuvre sera de préférence comprise entre 0,01 et 0,10 g/l.

Le milieu peut contenir également un antibiotique glycopeptide choisi dans le groupe constitue de la vancomycine, la teicoplanine et l'avoparcine et leurs mélanges.

Afin de détecter les microorganismes résistants à la fois à la méticilline et à la vancomycine. On peut utiliser environ de 5 mg/l à 50 mg/l de ces antibiotiques, plus particulièrement de 5 mg/l à 30 mg/l, de 10 mg/l à 30 mg/l, environ 25 mg/l.

Dans le cadre de la présente invention, les inventeurs ont montré qu'il n'est pas nécessaire d'avoir une forte charge osmotique dans le milieu de culture. Ainsi, contrairement aux milieux de détection de staphylocoques dorés résistants à la méticilline, utilisant l'oxacilline comme antibiotique, dans lesquels on ajoute du chlorure de sodium, le milieu de culture de l'invention est également fonctionnel avec une concentration de sodium inférieure à 3 %, et égale à environ 2-2,5 %. Les conditions d'incubation peuvent être adaptées en fonction de la quantité de chlorure de sodium dans le milieu (durée d'incubation, température plus ou moins élevée...).

L'invention se rapporte également à l'utilisation d'un milieu selon l'invention pour la détection de microorganismes résistants à la méticilline.

L'invention se rapporte également à un procédé de détection de microorganismes résistants à la méticilline dans un échantillon, comprenant les étapes consistant à :
- inoculer un milieu selon l'invention avec ledit échantillon ou un inoculum issu dudit échantillon,
- incuber ledit milieu dans des conditions permettant la croissance desdits microorganismes,
- détecter, sur ledit milieu, la présence desdits microorganismes résistants à la méticilline, par la présence de colonies colorées.

Les conditions d'incubation sont connues de l'homme du métier, et on utilise généralement une incubation à des températures comprises entre 25°C et 42°C, de préférence entre 30°C et 38°C.

Les durées d'incubation sont classiques (environ 24 heures).

Selon le microorganisme considéré, on peut utiliser une durée d'incubation plus courte ou plus longue, travailler dans des conditions aérobies ou anaérobies...

Le milieu selon l'invention permet notamment de détecter aisément les staphylocoques résistants à la méticilline, en réduisant le temps d'analyse. La combinaison des antibiotiques choisis dans le cadre de l'invention et des agents chromogènes permet en effet de réduire le nombre de faux positifs et de faux négatifs, et de diminuer ainsi le besoin de réaliser des analyses complémentaires.

### EXEMPLES

### Exemple 1

Composition d'un milieu selon l'invention pour la détection de *S. aureus* résistants à la méticilline :
Peptone et extrait de levure 40 g/l
NaCl 25 g/l
5-bromo 6-chloro 3-indoxyl phosphate 0,10 g/l
5-bromo 4-chloro 3-indoxyl glucoside 0, 05 g/l
5-bromo 4-chloro 3-indoxyl galactoside 0, 05 g/l
5-bromo 4-chloro 3-indoxyl glucuronide 0, 05 g/l
Déféroxamine 0,050 g/l
Agar 15 g/l.
On ajoute, dans ce milieu, de l'oxacilline (6 mg/ml) ou de la cefoxitine (5 mg/l), après autoclavage, avant que le milieu ne soit solide (lorsqu'il est à une température d'environ 45 °C).

Ce milieu contient du 5-bromo 6-chloro 3-indoxyl phosphate et du 5-bromo 4-chloro 3-indoxyl glucoside, permettant la détection spécifique des staphylocoques dorés (coloration mauve des colonies), ainsi que du 5-bromo 4-chloro 3-indoxyl galactoside et du 5-bromo 4-chloro 3-indoxyl glucuronide, pour colorer les autres microorganismes pouvant être présents dans l'inoculum.

### Exemple 2

Etude de la croissance de souches de *S*. *aureus* résistantes à la méticilline sur le milieu CHROMagar Staph aureus (disponible par la société CHROMagar, 4, Place du 18 Juin 1940, 75006 Paris France) :
AR4295 MetiS : souche sensible à la méticilline
AR4297 MetiR : souche résistante à la méticilline (homogène)
MRhet : souche résistante à la méticilline (hétérogène)
Z252 : souche résistante à la méticilline, homogène, faible niveau de résistance

| | AR4295 MetiS | AR4297 MetiR | MRhet | 2252 |
|---|---|---|---|---|
| CHROMagar Staph aureus | + | + | + | + |
| CHROMagar Staph aureus + oxacilline 6 mg/ml | - | +/-* | - | - |
| CHROMagar Staph aureus + Cefoxitine 5 mg/l | - | + | + | + |

| | | | | |
|---|---|---|---|---|
| + = croissance de colonies; - = pas de croissance ; * = microcolonies | | | | |

On inocule les boîtes de Pétri avec des cultures bactériennes en striant les boîtes, pour observer la croissance de bactéries isolées, après incubation à 37°C pendant 24 heures.

Les bactéries qui croissent donnent des colonies mauves sur le milieu de culture, confirmant que les microorganismes sont des staphylocoques dorés.

Ainsi, le milieu selon l'invention permet de détecter directement les staphylocoques dorés résistants à la méticilline, y compris les souches hétérogènes ou à faible niveau de résistance, du fait de la combinaison de la croissance des bactéries et de la coloration des colonies.

## Revendications

1. Milieu de culture gélosé pour la détection des *Staphylococcus aureus* résistants à la méticilline, comprenant, outre des nutriments permettant la croissance desdits *Staphylococcus aureus :*
- à titre d'antibiotique : une céphalosporine choisie dans le groupe constitué de la cefoxitine, le cefmetazole, le moxalactam et le flomoxef, et
- un agent chromogène libérant un chromophore après hydrolyse avec une enzyme active chez lesdits *Staphylococcus aurezis.*

2. Milieu selon la revendication 1, dans lequel ledit agent chromogène est le 5-bromo 6-chloro 3-indoxyl phosphate.

3. Milieu selon l'une des revendications 1 et 2, **caractérisé en ce qu'**il contient du 5-bromo 6-chloro 3-indoxyl phosphate et du 5-bromo 4-chloro 3-indoxyl glucoside.

4. Milieu selon l'une des revendications 1 à 3, **caractérisé en ce qu'**il comporte, en outre, au moins l'un des deux agents chromogènes suivants : 5-bromo 4-chloro 3-indoxyl galactoside et 5-bromo 4-chloro 3-indoxyl glucuronide.

5. Milieu selon l'une des revendications 1 à 4, **caractérisé en ce que la** concentration en chlorure de sodium est inférieure à 3 %.

6. Milieu selon l'une des revendications 1 à 5, **caractérisé en ce que la** concentration en antibiotique est comprise entre 0,5 et 50 mg/l.

7. Milieu selon l'une des revendications 1 à 6, **caractérisé en ce que la** concentration d'un agent chromogène est comprise entre 0,01 et 0,5 g/l.

8. Utilisation d'un milieu selon l'une des revendications 1 à 7 pour la détection des Staphylococcus aureus résistants à la méticilline.

9. Procédé de détection des *Staphylococcus aureus* résistants à la méticilline dans un échantillon, comprenant les étapes consistant à :
- inoculer un milieu selon l'une des revendications 1 à 7 avec ledit échantillon ou un inoculum issu dudit échantillon,
- incuber ledit milieu dans des conditions permettant la croissance desdits *Staphylococcus aureus,*
- détecter, sur ledit milieu, la présence desdits *Staphylococcus aureus* résistants à la méticilline, par la présence de colonies colorées.

## Claims

1. Agar culture medium for detecting meticillin-resistant *Staphylococcus aureus*, comprising, besides nutrients for the growth of said *Staphylococcus aureus:*
- as an antibiotic: a cephalosorin chosen from the group consisting of cefoxitin, cefmetazole, moxalactam and flomoxef, and
- a chromogenic agent that releases a chromophore after hydrolysis with an enzyme that is active in said *Staphylococcus aureus.*

2. Medium according to Claim 1, in which said chromogenic agent is 5-bromo-6-chloro-3-indoxyl phosphate.

3. Medium according to either of Claims 1 and 2, **characterized in that** it contains 5-bromo-6-chloro-3-indoxyl phosphate and 5-bromo-4-chloro-3-indoxyl glucoside.

4. Medium according to one of Claims 1 to 3, **characterized in that** it also comprises at least one of the following two chromogenic agents: 5-bromo-4-chloro-3-indoxyl galactoside and 5-bromo-4-chloro-3-indoxyl glucuronide.

5. Medium according to one of Claims 1 to 4, **characterized in that** the concentration of sodium chloride is less than 3%.

6. Medium according to one of Claims 1 to 5, **characterized in that** the concentration of antibiotic is between 0.5 and 50 mg/l.

7. Medium according to one of Claims 1 to 6, **characterized in that** the concentration of a chromogenic agent is between 0.01 and 0.5 g/l.

8. Use of a medium according to one of Claims 1 to 7, for detecting meticillin-resistant *Staphylococcus aureus*.

9. Method of detecting meticillin-resistant *Staphylococcus aureus* in a sample, comprising the steps consisting in:
- inoculating a medium according to one of Claims 1 to 7 with said sample or an inoculum derived from said sample,
- incubating said medium under conditions that allow growth of said *Staphylococcus aureus,*
- detecting, on said medium, the presence of said meticillin-resistant *Staphylococcus aureus* by virtue of the presence of coloured colonies.

## Patentansprüche

1. Mit Gelose Kulturmedium für den Nachweis von *Staphylococcus aureus,* welche gegen Meticillin resistent sind, umfassend außer den Nährstoffen, die die Vermehrung der *Staphylococcus aureus* erlauben:
- als Antibiotikum: ein Cephalosporin, welches in der aus Cefoxitin, Cefmetazol, Moxalactam und Flomoxef bestehenden Gruppe ausgewählt ist, und
- ein chromogenes Mittel, welches nach Hydrolyse mit einem in den *Staphylococcus aureus* aktiven Enzym ein Chromophor freisetzt.

2. Medium nach Anspruch 1, wobei das chromogene Mittel 5-Brom-6-chlor-3-indoxylphosphat ist.

3. Medium nach einem der Ansprüche 1 und 2, **dadurch gekennzeichnet, dass** es 5-Brom-6-chlor-3-indoxylphosphat und 5-Brom-4-chlor-3-indoxylglucosid enthält.

4. Medium nach einem der Ansprüche 1 bis 3, **dadurch gekennzeichnet, dass** es außerdem wenigstens eines der zwei folgenden chromogenen Mittel umfasst: 5-Brom-4-chlor-3-indoxylgalactosid und 5-Brom-4-chlor-3-indoxylglucuronid.

5. Medium nach einem der Ansprüche 1 bis 4, **dadurch gekennzeichnet, dass** die Natriumchlorid-Konzentration unter 3% liegt.

6. Medium nach einem der Ansprüche 1 bis 5, **dadurch gekennzeichnet, dass** die Antibiotika-Konzentration zwischen 0,5 und 50 mg/l eingeschlossen liegt.

7. Medium nach einem der Ansprüche 1 bis 6, **dadurch gekennzeichnet, dass** die Konzentration an einem chromogenen Mittel zwischen 0,01 und 0,5 g/l eingeschlossen liegt.

8. Verwendung eines Mediums nach einem der Ansprüche 1 bis 7 für den Nachweis von *Staphylococcus aureus,* welche gegen Meticillin resistent sind.

9. Verfahren zum Nachweis von *Staphylococcus aureus,* welche gegen Meticillin resistent sind, in einer Probe, welches die Schritte umfasst, welche daraus bestehen:
- ein Medium nach einem der Ansprüche 1 bis 7 mit der Probe oder einem Inokulum, welches von der Probe stammt, anzuimpfen,
- das Medium unter Bedingungen, welche die Vermehrung der *Staphylococcus aureus* erlauben, zu inkubieren,
- auf dem Medium das Vorhandensein der *Staphylococcus aureus,* welche gegen Meticillin resistent sind, durch das Vorhandensein von gefärbten Kolonien nachzuweisen.
